# EUROPÄISCHE PATENTSCHRIFT

(11) **EP 0 969 811 B1**
(45) Veröffentlichungstag und Bekanntmachung des Hinweises auf die Patenterteilung: **16.01.2002**
(21) Anmeldenummer: 98910689.3
(22) Anmeldetag: 17.02.1998
(51) Int. Cl.: A61K 7/50

(54) **MISCHUNGEN VON ALKYLPHOSPHORSÄUREESTERN UND DEREN VERWENDUNG ALS KOSMETISCHE UND PHARMAZEUTISCHE EMULGATOREN**
MIXTURES OF ALKYLPHOSPHORIC ACID ESTERS AND THEIR USE AS COSMETIC AND PHARMACEUTICAL EMULSIFIERS
MELANGES D'ESTERS D'ACIDE PHOSPHORIQUE D'ALKYLE ET LEURS UTILISATION COMME EMULSIFIANTS COSMETIQUES ET PHARMACEUTIQUES

(30) Priorität: 27.02.1997 DE 19707800
(43) Veröffentlichungstag der Anmeldung: 12.01.2000
(73) Patentinhaber: Clariant GmbH, 65929 Frankfurt am Main (DE)
(72) Erfinder: TUROWSKI-WANKE, Angelika, D-65777 Kelkheim (DE); LÖFFLER, Matthias, D-65527 Niedernhausen (DE); EYRISCH, Oliver, D-45127 Essen (DE); SKRYPZAK, Werner, D-65719 Hofheim (DE)
(86) Internationale Anmeldenummer: EP9800890
(87) Internationale Veröffentlichungsnummer: WO9837867

(56) Entgegenhaltungen:
- EP-A- 0 347 844
- EP-A- 0 442 701
- DE-A- 2 744 980
- GB-A- 2 139 112
- US-A- 4 101 457
- US-A- 4 247 424

## Beschreibung

Diese Erfindung betrifft Mono-, Di- und in geringen Mengen auch Trialkylphosphorsäureestern oder deren Salzen, bevorzugt auf Basis von β-verzweigten Fettalkoholen, besonders bevorzugt auf Basis von Guerbetalkohol, mit sehr effektiver Wirkung auf die Erniedrigung der Grenzflächenspannung von sowohl polaren als auch unpolaren Flüssigkeiten, hoher Stabilität auch bei höheren Temperaturen und geringer Empfindlichkeit gegenüber Elektrolyten und Säuren.

Die Erfindung betrifft weiterhin die Verwendung solcher Mischungen als Emulgatoren in kosmetischen und pharmazeutischen Formulierungen, sowie die so hergestellten Erzeugnisse.

Die Verwendung von Emulgatoren zur Herstellung von Cremes, Lotionen, Salben etc., die mehrere nicht miteinander mischbare Substanzen (z.B. Wasser, Öl, organische und anorganische Bestandteile) enthalten, ist lange bekannt. Als Emulgatoren wirken Tenside, z.B. Seifen von Alkalimetallen und Alkanolaminen, Mono- und Diglycerinester von Fettsäuren, aber auch bestimmte Naturstoffe (z.B. Lecithine, Wachse) und anorganische Stoffe (z.B. Bentonite).

Aus EP-B-0 553 241 ist die Verwendung von Mischungen aus Alkyloligoglucosiden, Fettalkoholen und gegebenenfalls Polyglucose zur Herstellung von Emulsionen bekannt. In WO-92/ 07543 wird der Einsatz von Alkyloligoglucosiden mit Fettalkoholen und Partialglyceriden als kosmetische Emulgatoren offenbart.

In EP-B-0 201 040 wird das Emulgiervermögen von Metallsalzen von Dialkylphosphaten und in EP-B-0 227 012 das von Mono-Phosphorsäureestem beschrieben.

GB-A-2 139 112 offenbart eine Emulgatormischung bestehend aus Mono- und Diesterphosphorsäure, teilweise auch ethoxiliert, im Verhältnis 100 : 0 bis 70 : 30 sowie einem nichtionischen Tensid.

Der Einsatz von β-verzweigten Monoalkylphosphorsäureestern als Emulgator wird in EP-A-0 265 702 offenbart.

DE-A-2 744 980 und US-A-4 101 457 offenbaren Zusammensetzungen, welche Mono- , Di- und Trialkylphosphate enthalten.

Die genannten Emulgatoren weisen jedoch auch nachteilige Eigenschaften wie geringe Stabilität bei höheren Temperaturen, eingeschränkte Mischbarkeit oder Löslichkeit gegenüber wäßrigen bzw. organischen Medien und Verminderung des Emulgiervermögens durch Zusatz von salzartigen Komponenten auf.

Die Aufgabe bestand somit darin, leistungsstarke, stabile, gegenüber salzartigen Komponenten, beispielsweise Elektrolyten unempfindliche Emulgatoren zu entwickeln. Aus anwendungstechnischen Gründen sollten diese bei Raumtemperatur flüssig sein und zur Gewährleistung einer guten Hautverträglichkeit einen pH-Wert von ca. 5 bis 7 haben.

Überraschenderweise wurde gefunden, daß Mischungen aus Mono-, Di- und Trialkylmonophosphorsäureestern die geforderten Eigenschaften aufweisen.

Gegenstand der Erfindung sind Mischungen aus Mono-, Di- und Trialkylmonophosphorsäureestem gemäß den Formeln I, II und III 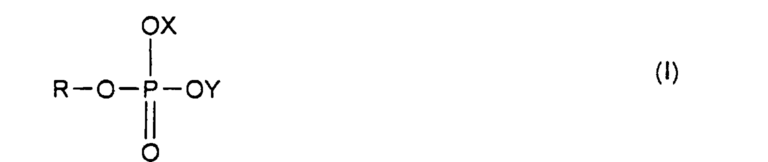 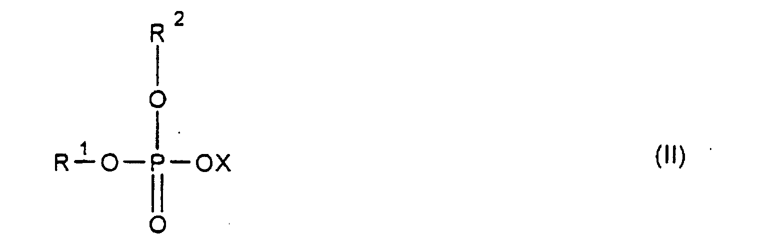 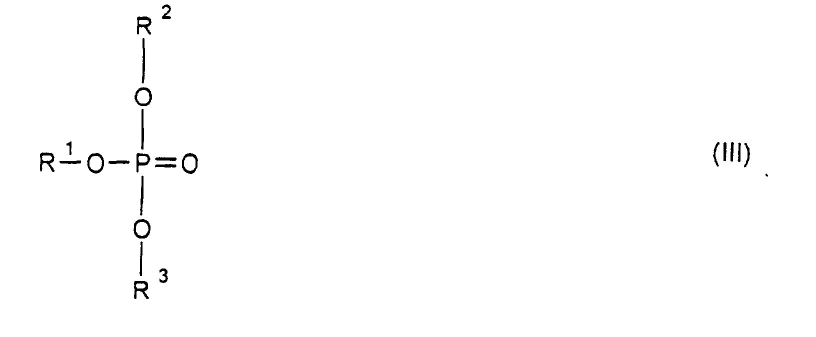 worin
R, R¹, R² und R³, die gleich oder verschieden sein können, beta - verzweigte Alkylgruppen mit 16 bis 20 Kohlenstoffatomen, und
X und Y, die gleich oder verschieden sein können, Wasserstoff, Alkalimetall oder Erdalkalimetall, substituiertes oder unsubstituiertes Ammonium oder organische basische Gruppen bedeuten.

Organische basische Gruppen unter der Bedeutung von X und Y sind beispielsweise basische Aminosäuren, wie etwa Arginin, Ornithin, Lysin, sowie Alkanolamine z.B. Triethanolamin oder Monoethanolamin. Beispiele für substituierte Ammoniumkationen sind C₈-C₂₂-Alkylammonium-lonen.

Die Erfindung betrifft weiterhin die Verwendung dieser Mischungen von Alkylphosphorsäureestern als Emulgatoren in kosmetischen oder pharmazeutischen Formulierungen sowie kosmetische oder pharmazeutische Formulierungen, die diese Ester enthalten.

Die erfindungsgemäßen Mischungen enthalten sowohl die Monoester der Formel I als auch die Diester der Formel II jeweils in Mengen von ca.30 bis 60, insbesondere von ca. 40 bis 50 Gew.-%. Die Triester der Formel III sind in Mengen zwischen ca. 0,5 und 5, bevorzugt von ca. 1 bis 2 Gew.-% in den erfindungsgemäßen Mischungen enthalten.

Die Phosphorsäureestermischung wird üblicherweise in Mengen von 0,1 bis 5 Gewichtsprozent, bevorzugt in Mengen von 0,3 bis 3 Gewichtsprozent, bezogen auf fertige Formulierungen, eingesetzt.

Es wurde gefunden, daß Mischungen aus Phosphorsäureestern, zusammengesetzt aus Mono-, Di- und Triester gemäß den Formeln I, II und III dann herausragende Emulgatoreigenschaften aufweisen, wenn R, R¹, R² und R³ β-verzweigte Alkylgruppen mit 16 bis 20 C-Atomen sind. Die diesen Resten zugrunde liegenden Alkohole werden als Guerbetalkohole bezeichnet. Alkylphosphorsäureester mit β-verzweigten Alkylgruppen mit 16 bis 20 C-Atomen für R, R¹, R² und R³ sind der Gegenstand dieser Erfindung.

Die Darstellung der Alkylphosphorsäureester erfolgt durch Umsetzung von Tetraphosphordecaoxid und Alkylfettalkoholen unter Bildung von Mono- und Diester im ungefähren Molverhältnis 1:1 mit geringen Anteilen an Triester, wie in der Reaktionsgleichung dargestellt (R steht hier für alle Reste R, R¹, R² und R³):

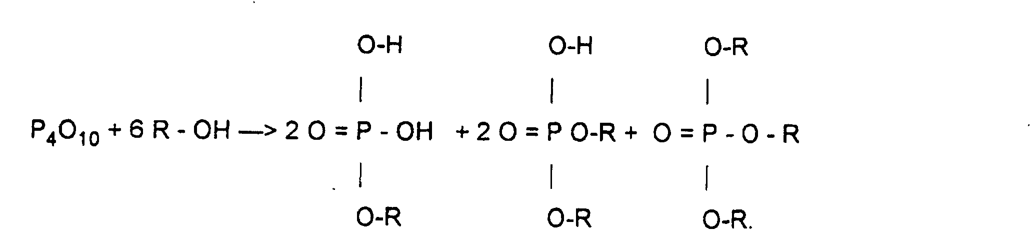

Die Alkylphosphorsäureestermischungen sind gelbliche bis weiße, teils feste oder wachsartige, aber viskose und flüssige Substanzen mit Schmelzpunkten zwischen-5°C und 80°C und Jodfarbzahlen von < 4 bis < 0,5.

Setzt man P₄O₁₀ mit C₁₆/C₂₀- Guerbetalkohol um, so erhält man ein Phosphorsäureestergemisch mit R, R¹, R² und R³ = C₁₆-C₂₀-β-verzweigtes Alkyl mit besonders vorteilhaften physikalisch-chemischen Kennzahlen. Die hellgelbe, flüssige Mischung, bestehend aus ca. 40-60 Gew.-% Mono-, ca. 30-50 Gew-% Di- und bis zu 10 Gew-% Triester, hat einen Schmelzpunkt von < -5 °C, eine Jodfarbzahl von < 1,0 und weist eine gute Löslichkeit sowohl in polaren als auch in unpolaren organischen Substanzen, wie Paraffin, Sojaöl und Isopropylpalmitat auf.

Die erfindungsgemäßen Phosphorsäureestergemische, vorzugsweise solche, worin R, R¹, R² und R³ β-verzweigtes C₁₆-C₂₀-Alkyl bedeutet, sind insbesondere zur Herstellung von Öl-in-Wasser-Emulsionen, aber auch von Wasser-in-Öl-Emulsionen geeignet, bevorzugt zur Herstellung alkoholfreier Emulsionen.

Die Leistungsstärke eines Emulgators korreliert mit der Erniedrigung der Grenzflächenspannung. Phosphorsäureester der oben beschriebenen Zusammensetzung zeichnen sich durch grenzflächenspannungserniedrigende Wirkung aus. Überraschenderweise wurde gefunden, daß Phosphorsäureester auf Basis von Guerbetalkoholen eine drastische Absenkung der Grenzflächenspannung bewirken, was die Leistungsstärke als Emulgator widerspiegelt.

**Tabelle 1:**

| Grenzflächenspannungsverhalten verschiedener Phosphorsäureester gegenüber Ölen unterschiedlicher Polarität | | | |
|---|---|---|---|
| Phosphorsäureester | Paraffinöl | Cetearylisononanoat | Sojaöl |
| Lauryl | 28 | 16.5 | 16 |
| Stearyl | 16 | 15.8 | 15.5 |
| Isostearyl | 11 | 8.5 | 9.0 |
| Isooctadecyl | 1.5 | 2.5 | 3.0 |
| Oleyl | 13 | 11 | 9.0 |
| Behenyl | 36 | 24 | 17.5 |

Grenzflächenspannung in mN/m
Temperatur 25°C, Konzentration 1,5 g Phosphorsäureester in 1 Liter Wasser, pH 7, Na-Salz
"Cetearyl" bezeichnet eine Mischung aus Cetyl und Stearyl.

Die Bestimmung der Grenzflächenspannung erfolgte in einem Lauda Tropfenvolumen Tensiometer.

Der Begriff "Phosphorsäureester" in dieser Tabelle bedeutet eine erfindungsgemäße Mischung aus ungefähr gleichen Teilen Mono- und Di-ester sowie einem geringen Anteil Tri-ester.

Stabilitätstests mit verschiedenen Ölen, wie Squalan, Sojaöl, Cetearylisononanoat und Isopropylpalmitat ergaben, daß Emulsionen, die die Phosphorsäureester auf Basis von Guerbetalkoholen enthalten im 30-Tage-Lagertest bei 40, 45 und 50°C keinerlei Entmischung zeigen. Vergleichbare Ergebnisse konnten mittels Zentrifugentest bei verschiedenen Emulgatorkonzentrationen erhalten werden.

Die erfindungsgemäßen Emulgatoren zeichnen sich durch eine starke grenzflächenspannungserniedrigende Wirkung auch bei hoher Temperaturbelastung gegenüber polaren und unpolaren Bestandteilen aus. Die zum Teil flüssigen Emulgatoren zeigen eine verbesserte Stabilität gegenüber Elektrolytzusätzen und Säuren und eine hohe Lagerstabilität. Sie liegen im pH-Bereich 5 bis 7 vor und können somit als sehr hautfreundliche Emulgatoren eingesetzt werden, bevorzugt in Hautpflegemitteln.

Der nichtwäßrige Anteil der Emulsionen, der sich weitgehend aus dem Emulgator- und dem Ölkörper zusammensetzt und dabei in der Regel dem Feststoffgehalt entspricht, liegt üblicherweise bei 5 bis 95 % und vorzugsweise 15 bis 75 Gew.-%. Das bedeutet, daß die Emulsionen 5 bis 95 und vorzugsweise 25 bis 85 Gew.-% Wasser enthalten können, abhängig davon, ob Lotionen mit einer vergleichsweise niedrigen oder Cremes und Salben mit hoher Viskosität hergestellt werden sollen.

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₁₃ Fettsäuren mit linearen C₆-C₂₀ Fettalkoholen, Ester von verzweigten C₆-C₁₃ Carbonsäuren mit linearen C₆-C₂₀-Fettalkoholen, Ester von linearen C₆-C₁₈-Fettsäuren mit verzweigten Alkoholen, insbesondere 2-Ethylhexanol, Ester von linearen und/oder verzweigten Fettsäuren mit mehrwertigen Alkoholen (wie z.B. Dimerdiol oder Trimerdiol) und/oder Guerbetalkoholen, Triglyceride auf Basis C₆-C₁₀-Fettsäuren, pflanzliche Öle, verzweigte primäre Alkohole, substituierte Cyclohexane, Guerbetcarbonate, Dialkylether und/oder aliphatische bzw. aromatische Kohlenwasserstoffe in Betracht. Der Anteil der Ölkörper am nichtwäßrigen Anteil der Emulsionen kann 5 bis 95 und vorzugsweise 15 bis 75 Gew.-% ausmachen. .

Die Emulsionen können als Hautpflegemittel, wie beispielsweise Tagescremes, Nachtcremes, Pflegecremes, Nährcreme, Bodylotions, Salben und dergleichen eingesetzt werden und als weitere Hilfs- und Zusatzstoffe, Co-Emulgatoren, Überfettungsmittel, Fette, Wachse, Stabilisatoren, biogene Wirkstoffe, Glycerin, Konservierungsmittel, Farb- und Duftstoffe enthalten.

Wesentlich für die Erfindung ist, daß die beschriebenen Mischungen der Phosphorsäureester auch ohne Mitverwendung eines nicht-ionischen Tensids als Co-Emulgator eingesetzt werden können. Die Mitverwendung von Co-Emulgatoren ist daher nicht zwingend, aber möglich.

Als nichtionogene O/W-Co-Emulgatoren kommen in Betracht Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe; C₁₂-C₁₈-Fettsäuremono- und -diester von Anlagerungsprodukten von 1 bis 30 Mol Ethylenoxid an Glycerin; Glycerinmono- und -diester und Sorbitanmono- und -diester von gesättigten und ungesättigten Fettsäuren mit 6 bis 22 Kohlenstoffatomen und deren Ethylenoxidanlagerungsprodukten; Anlagerungsprodukte von 15 bis 60 Mol Ethylenoxid an Rizinusöl und/oder gehärtetes Rizinusöl; Polyol- und insbesondere Polyglycerinester wie z.B. Polyglycerinpolyricinoleat und Polyglycerinpoly-12-hydroxystearat. Ebenfalls geeignet sind Gemische von Verbindungen aus mehreren dieser Substanzklassen. Die Anlagerungsprodukte von Ethylenoxid und/oder Propylenoxid an Fettalkohole, Fettsäuren, Alkylphenole, Glycerin-mono- und - diester sowie Sorbitanmono- und -diester von Fettsäuren oder an Rizinusöl stellen bekannte, im Handel erhältliche Produkte dar. Es handelt sich dabei um Homologengemische, deren mittlerer Alkoxilierungsgrad dem Verhältnis der Stoffmengen von Ethylenoxid und/oder Propylenoxid und Substrat, mit denen die Anlagerungsreaktion durchgeführt wird, entspricht. C₁₂-C₁₈-Fettsäuremono- und - diester von Anlagerungsprodukten von Ethylenoxid an Glycerin sind aus DE-20 24 051 als Rückfettungsmittel für kosmetische Zubereitungen bekannt.

Als Überfettungsmittel können Substanzen wie beispielsweise polyethoxilierte Lanolinderivate, Lecithinderivate, Polyolfettsäureester, Monoglyceride und Fettsäurealkanolamide verwendet werden, wobei die letzteren gleichzeitig als Schaumstabilisatoren dienen. Typische Beispiele für Fette sind Glyceride, als Wachse kommen u.a. Bienenwachs, Paraffinwachs oder Mikrowachse, gegebenfalls in Kombination mit hydrophilen Wachsen, z.B. Cetylstearylalkohol in Frage. Als Stabilisatoren können Metallsalze von Fettsäuren wie z.B. Magnesium-, Aluminium- und/oder Zinkstearat eingesetzt werden. Unter biogenen Wirkstoffen sind beispielsweise Pflanzenextrakte und Vitaminkomplexe zu verstehen. Als Konservierungsmittel eignen sich beispielsweise Phenoxyethanol, Formaldehydlösung, Parabene, Pentandiol oder Sorbinsäure. Als Perlglanzmittel kommen beispielsweise Glycoldistearinsäureester wie Ethylenglycoldistearat, aber auch Fettsäuremonoglycolester in Betracht. Als Farbstoffe können die für kosmetische Zwecke geeigneten und zugelassenen Substanzen verwendet werden, wie sie beispielsweise in der Publikation "Kosmetische Färbemittel" der Farbstoffkommission der Deutschen Forschungsgemeinschaft, Verlag Chemie, Weinheim, 1984, S.81-106 zusammengestellt sind.

Der Gesamtanteil der Hilfs- und Zusatzstoffe kann 1 bis 10, vorzugsweise 2 bis 5 Gew.-% bezogen auf das Mittel, betragen.
Die Herstellung der Mittel kann in an sich bekannter Weise, d.h. beispielsweise durch Heiß-, Heiß-Heiß/ Kalt- bzw. PIT- Emulgierung erfolgen.

### Beispiele

### O/W-Hautmilch

"Ethylenoxidfrei", kalt herstellbar

### Zusammensetzung

| | | | |
|---|---|---|---|
| A | ®Hostaphat VCG 120 | (Hoechst AG) | 1,50 % |
| | Paraffinöl, niedrigviskos | | 5,00 % |
| | ®Miglyol 812 | (Dynamit Nobel) | 4,00 % |
| | Isopropylpalmitat | | 6,00 % |
| | Jojobaöl | | 2,00 % |
| | Sojaöl | | 3,00 % |
| | | | |
| B | ®Carbopol 980 | (Goodrich) | 0,50 % |
| | | | |
| C | ®Aquamollin BC Plv. Hochkonz. | (Hoechst AG) | 0,10 % |
| | Citronensäure (10 %ig) | | 0,30 % |
| | NaOH (10 %ig) | | 3,20 % |
| | Glycerin | | 3,00% |
| | Wasser | | 71.0 % |
| | Konservierungsmittel | | q.s. |
| | | | |
| D | Paraffinöl | | 0.30 % |

### Herstellung

I B in A eintragen, anschließend C einrühren und gut verrühren
II D in I einrühren
III Abschließend die Emulsion homogenisieren

### O/W-Creme

"Ethylenoxidfrei", Mineralölfrei

### Zusammensetzung

| | | | |
|---|---|---|---|
| A | ®Hostaphat VCG 120 | (Hoechst AG) | 1,00 % |
| | ®Hostacerin DGMS | (Hoechst AG) | 2,50 % |
| | ®Miglyol 812 | (Dynamit Nobel) | 3,00 % |
| | ®Cetiol SN | (Henkel KGaA) | 4,00 % |
| | Isopropylpalmitat | | 5,00 % |
| | Weizenkeimöl | | 1,00 % |
| | Jojobaöl | | 3,00 % |
| | Sojaöl | | 4,00 % |
| | | | |
| B | ®Carbopol 980 | (Goodrich) | 0,60 % |
| | | | |
| C | ®Hostapon KCG | (Hoechst AG) | 0,60 % |
| | ®Aquamollin BC Plv. Hochkonz. | (Hoechst AG) | 0,10 % |
| | Citronensäure (10 %ig) | | 0,30 % |
| | NaOH (10 %ig) | | 2,90 % |
| | Glycerin | | 3,00 % |
| | Wasser | | 68.60 % |
| | Konservierungsmittel | | q.s. |
| | | | |
| D | Parfümöl | | 0,40 % |

### Herstellung

I A bei ca. 80°C aufschmelzen, anschließend B eintragen
II C auf ca. 80°C erwärmen
III II unter Rühren I zusetzen und kalt rühren
IV Bei ca. 35°C in III einrühren
V Abschließend die Emulsion homogenisieren.

### O/W-Hautmilch

"Ethylenoxidfrei"; kalt herstellbar

### Zusammensetzung

| | | | |
|---|---|---|---|
| A | ®Hostaphat VCG 120 | (Hoechst AG) | 1,50 % |
| | Isopropylpalmitat | | 6,00 % |
| | Paraffinöl, niedrigviskos | | 5,00 % |
| | ®Miglyol 812 | (Dynamit Nobel) | 4,00 % |
| | Sojaöl | | 3,00 % |
| | Jojobaöl | | 2,00 % |
| | | | |
| B | ®Carbopol 980 | (Goodrich) | 0,50 % |
| | | | |
| C | ®Aquamollin BC Plv. hochkonz. | (Hoechst AG) | 0,10 % |
| | Citronensäure (10 %ig) | | 0,30 % |
| | Glycerin | | 3,00 % |
| | Wasser | | 71,10% |
| | Natronlauge (10 %ig) | | 3,20% |
| | Konservierungsmittel | | q.s. |
| | | | |
| D | Parfümöl | | 0,30 % |

### Herstellung

I B in A eintragen, anschließend C einrühren und gut verrühren
II D in I einrühren
III Abschließend die Emulsion homogenisieren.

### O/W-Creme

Ethylenoxidfrei

### Zusammensetzung

| | | | |
|---|---|---|---|
| A | ®Hostaphat VCG 120 | (Hoechst AG) | 1,00 % |
| | ®Hostacerin DGMS | (Hoechst AG) | 2,50 % |
| | Isopcopylpalmitat | | 6,00 % |
| | Paraffinöl, niedrigviskos | | 5,00 % |
| | ®Miglyol 812 | (Dynamit Nobel) | 4,00 % |
| | Sojaöl | | 3,00 % |
| | Jojobaöl | | 2,00 % |
| | | | |
| B | ®Carbopol 980 | (Goodrich) | 0,60 % |
| | | | |
| C | ®Aquamollin BC Plv. hochkonz. | (Hoechst AG) | 0,10 % |
| | Citronensäure (10 %ig) | | 0,30 % |
| | Glycerin | | 3,00 % |
| | Wasser | | 68,90 % |
| | Natronlauge (10 %ig) | | 3,20 |
| | Konservierungsmittel | | q.s. |
| | | | |
| D | Parfümöl | | 0,40 % |

### Herstellung

I A bei ca. 60°C aufschmelzen, anschließend B eintragen.
II C auf ca. 60°C erwärmen
III II unter Rühren I zusetzen und kalt rühren
IV Bei ca. 35°C D in III einrühren
V Abschließend die Emulsion homogenisieren

### O/W-Hautmilch

Ethylenoxidfrei, mineralölfrei

### Zusammensetzung

| | | | |
|---|---|---|---|
| A | ®Hostaphat VCG 120 | (Hoechst AG) | 1,00 % |
| | ®Hostacerin DGMS | (Hoechst AG) | 1,50 % |
| | Isopropylpalmitat | | 5,00 % |
| | ®Cetiol SN | (Henkel KGaA) | 4,00 % |
| | Sojaöl | | 4,00 % |
| | ®Miglyol 812 | (Dynamit Nobel) | 3,00 % |
| | Jojobaöl | | 3,00 % |
| | Weizenmehl | | 1,00 % |
| | | | |
| B | ®Carbopol 980 | (Goodrich) | 0,60 % |
| | | | |
| C | ®Aquamollin BC Plv. hochkonz. | (Hoechst AG) | 0,10 % |
| | Citronensäure (10 %ig) | | 0,30 % |
| | Glycerin | | 3,00 % |
| | Wasser | | 71,00% |
| | Natronlauge (10 %ig) | | 2,40% |
| | Konservierungsmittel | | q.s. |
| | | | |
| D | Parfümöl | | 0,30 % |

### Herstellung

I A bei ca. 60°C aufschmelzen, anschließend B eintragen.
II C auf ca. 60°C erwärmen
III II unter Rühren I zusetzen und kalt rühren
IV Bei ca. 35°C in III einrühren
V Abschließend die Emulsion homogenisieren.

### O/W-Hautmilch

Ethylenoxidfrei

### Zusammensetzung

| | | | |
|---|---|---|---|
| A | ®Hostaphat VCG 120 | (Hoechst AG) | 1,00 % |
| | ®Hostacerin DGMS | (Hoechst AG) | 2,00 % |
| | Isopropylpalmitat | | 6,00 % |
| | Paraffinöl, niedrigviskos | | 5,00 % |
| | ®Miglyol 812 | (Dynamit Nobel) | 4,00 % |
| | Sojaöl | | 3,00 % |
| | Jojobaöl | | 2,00 % |
| | | | |
| B | ®Carbopol 980 | (Goodrich) | 0,40 % |
| | | | |
| C | ®Aquamollin BC PIv. hochkonz. | (Hoechst AG) | 0,10 % |
| | Ethylendiamintetraessigsäure-Natriumsalz Citronensäure (10 %ig) | | 0,30 % |
| | Glycerin | | 3,00 % |
| | Wasser | | 71,00% |
| | Natronlauge (10 %ig) | | 2,40% |
| | Konservierungsmittel | | q.s. |
| | | | |
| D | Parfümöl | | 0,30 % |

### Herstellung

I A bei ca. 60°C aufschmelzen, anschließend B eintragen.
II C auf ca. 60°C erwärmen
III II unter Rühren I zusetzen und kalt rühren
IV Bei ca. 35°C in III einrühren
V Abschließend die Emulsion homogenisieren.

### O/W-Hautmilch

kalt herstellbar

### Zusammensetzung

| | | | |
|---|---|---|---|
| A | ®Hostaphat VCG 120 | (Hoechst AG) | 1,50 % |
| | ®Hostacerin DGI | (Hoechst AG) | 2,00 % |
| | Paraffinöl, niedrigviskos | | 8,00 % |
| | Isopropylpalmitat | | 6,00 % |
| | ®Cetiol 868 | (Henkel KGaA) | 5,00 % |
| | | | |
| B | ®Carbopol 980 | (Goodrich) | 0,40 % |
| | | | |
| C | Wasser | | 75,20 % |
| | Natronlauge (10 %ig) | | 1,60 % |
| | Konservierungsmittel | | q.s. |
| | | | |
| D | Parfümöl | | 0,30 % |

### Herstellung

I B in A eintragen, anschließend C einrühren
II D in I einrühren
III Abschließend die Emulsion homogenisieren.

### O/W-Feuchtigkeitsmilch

### Zusammensetzung

| | | | |
|---|---|---|---|
| A | ®Hostaphat VCG 120 | (Hoechst AG) | 1,00 % |
| | ®Hostacerin DGS | (Hoechst AG) | 4,00 % |
| | Isopropylisostearat | | 4,00 % |
| | ®Cetiol V | (Henkel KGaA) | 4,00 % |
| | Walnußöl | | 4,00 % |
| | Paraffinöl, niedrigviskos | | 3,00 % |
| | Antioxydans | | q.s. |
| | | | |
| B | ®Carbopol 980 | (Goodrich) | 0,30 % |
| | | | |
| C | ®Aquamollin BC PIv. hochkonz. | (Hoechst AG) | 0,10 % |
| | Citronensäure (10 %ig) | | 0,25% |
| | Glycerin | | 3,00 % |
| | Wasser | | 69,85 % |
| | Natronlauge (10 %ig) | | 1,20 % |
| | Konservierungsmittel | | q.s. |
| | | | |
| D | Parfümöl | | 0,30 % |

### Herstellung

I A bei ca. 70°C aufschmelzen, anschließend B eintragen
II C auf ca. 70°C erwärmen.
III II unter Rühren I zusetzen und kalt rühren.
IV Bei ca. 35°C D in III einrühren.
V Abschließend die Emulsion homogenisieren.

Es wurden folgende Handelsprodukte verwendet:
®Carbopol 980
   Acrylsäure-Polymerisate, die in einem Gemisch aus Ethylacetat und Cyclohexan polymerisiert werden.
®Miglyol 812
   Capryltriglycerid
®Aquamollin BC Plv. Hochkonz.
   Ethylendiamintetraessigsäure-Natriumsalz
®Hostacerin DGMS
   Polyglyceryl-2-stearat
®Cetiol SN
   Cetearylisononanoat
®Cetiol 868
   Isooctylstearat
®Cetiol V
   Decyloleat
®Hostapon KCG
   Natriumcocoylglutamat
®Hostaphat VCG 120
   Mono/Di-Phosphorsäureester auf Basis von C₁₈-Guerbetalkohol
®Hostacerin DGI
   Polyglyceryl-2-sesquiisostearat

## Patentansprüche

1. Mischungen aus Mono-, Di- und Trialkylmonophosphorsäureestern gemäß den Formeln I, II und III 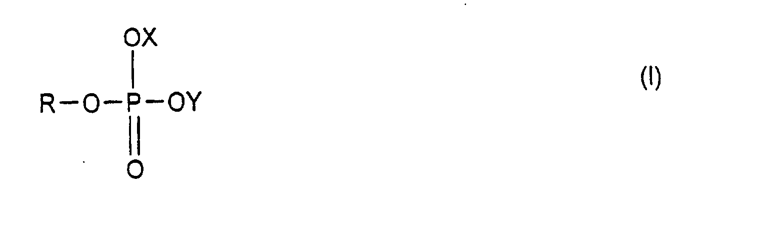 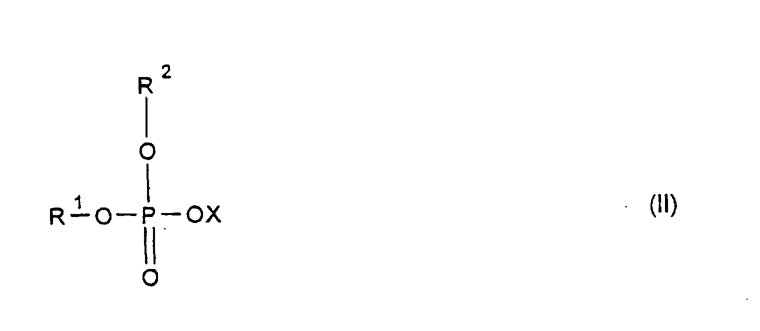 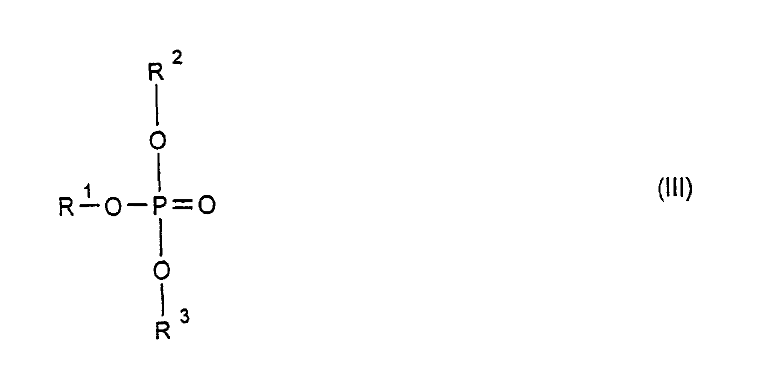 worin
R, R¹, R² und R³, die gleich oder verschieden sein können, beta-verzweigte Alkylgruppen mit 16 bis 20 Kohlenstoffatomen, und
X und Y, die gleich oder verschieden sein können, Wasserstoff, Alkalimetall oder Erdalkalimetall, substituiertes oder unsubstituiertes Ammonium oder organische basische Gruppen bedeuten.

2. Mischungen nach Anspruch 1, **dadurch gekennzeichnet, daß** es sich bei den organischen basischen Gruppen unter der Definition von X und Y um basische Aminosäuren, wie Arginin, Ornithin, Lysin, sowie Alkanolamine handelt.

3. Mischungen nach den Ansprüchen 1 und/oder 2, **dadurch gekennzeichnet, daß** sie die Monoester der Formel I und die Diester der Formel II in Mengen von 30 bis 60, insbesondere von 40 bis 50 Gew.-% enthalten.

4. Mischungen nach einem oder mehreren der Ansprüche 1 bis 3, **dadurch gekennzeichnet, daß** sie die Triester der Formel III in Mengen zwischen 0,5 und 5, bevorzugt von 1 bis 2 Gew.-% enthalten.

5. Kosmetische und/oder pharmazeutische Formulierungen, **dadurch gekennzeichnet, daß** sie Mischungen gemäß einem oder mehreren der Ansprüche 1 bis 5 in Mengen von 0,1 bis 5, bevorzugt 0,3 bis 3 Gew.-%, bezogen auf das Gewicht der Formulierung enthalten.

6. Verwendung der Mischungen nach einem oder mehreren der Ansprüche 1 bis 5 als Emulgator kosmetischer und pharmazeutischer Erzeugnisse.

7. Verwendung der Mischungen nach einem oder mehreren der Ansprüche 1 bis 5 zusammen mit Co-Emulgatoren.

8. Verwendung der Mischungen nach einem oder mehreren der Ansprüche 1 bis 5 als Emulgator von Öl-in-Wasser-Emulsionen.

9. Verwendung der Mischungen nach einem oder mehreren der Ansprüche 1 bis 5 als Emulgator in Hautpflegemitteln.

## Claims

1. A mixture of mono-, di- and trialkylmonophosphoric esters of the formulae I, II and III 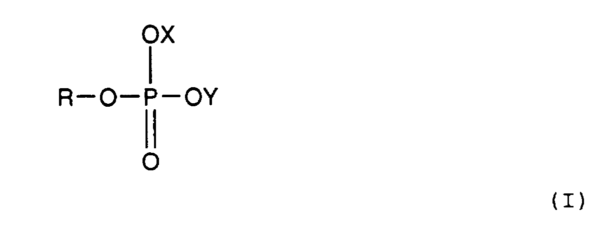 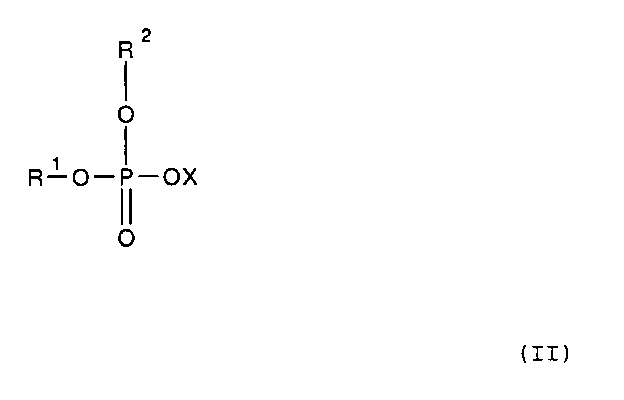 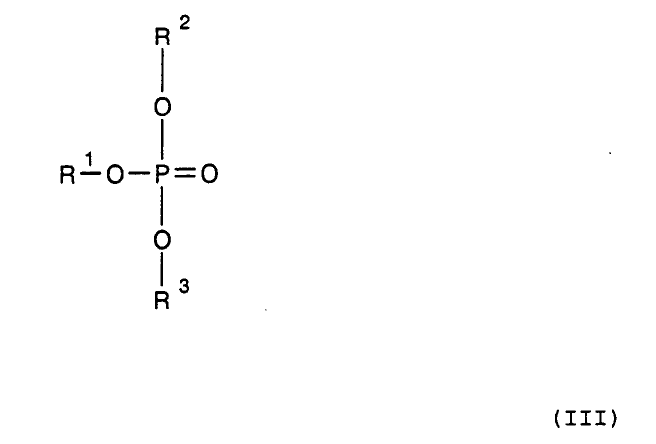 in which
R, R¹, R² and R³, which may be identical or different, are beta-branched alkyl groups having from 16 to 20 carbon atoms, and
X and Y, which may be identical or different, are hydrogen, alkali metal or alkaline earth metal, substituted or unsubstituted ammonium or basic organic groups.

2. The mixture as claimed in claim 1, wherein the basic organic groups under the definition of X and Y are basic amino acids, such as arginine, ornithine and lysine, or alkanolamines.

3. The mixture as claimed in claims 1 and/or 2, which comprises the monoesters of the formula I and the diesters of the formula II in amounts of from 30 to 60% by weight, in particular from 40 to 50% by weight.

4. The mixture as claimed in one or more of claims 1 to 3, which comprises the triesters of the formula III in amounts of between 0.5 and 5% by weight, preferably from 1 to 2% by weight.

5. A cosmetic and/or pharmaceutical formulation which comprises the mixture as claimed in one or more of claims 1 to 4 in amounts of from 0.1 to 5% by weight, preferably from 0.3 to 3% by weight, based on the weight of the formulation.

6. The use of the mixture as claimed in one or more of claims 1 to 4 as an emulsifier in cosmetic and pharmaceutical products.

7. The use of the mixture as claimed in one or more of claims 1 to 4 together with coemulsifiers.

8. The use of the mixture as claimed in one or more of claims 1 to 4 as an emulsifier of oil-in-water emulsions.

9. The use of the mixture as claimed in one or more of claims 1 to 4 as an emulsifier in skincare products.

## Revendications

1. Mélanges d'esters mono-, di- et trialkyliques d'acide monophosphorique selon les formules I, II, et III 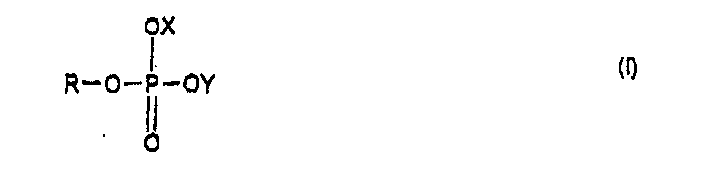 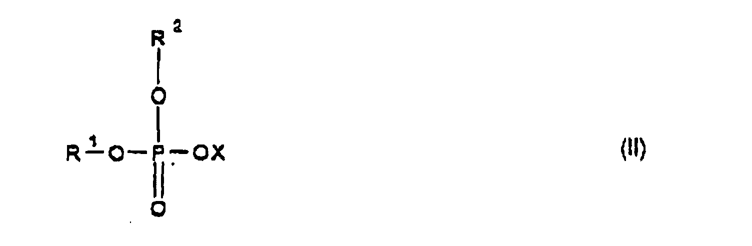 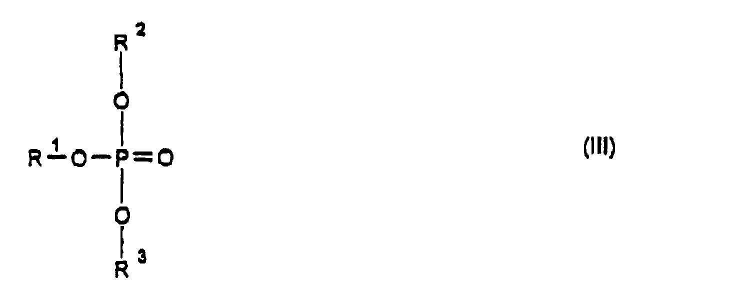 dans lesquelles
R, R¹, R² et R³, qui peuvent être identiques ou différents, représentent des groupes alkyle ayant de 16 à 20 atomes de carbone ramifiés en bêta, et
X et Y, qui peuvent être identiques ou différents, représentent un atome d'hydrogène, un métal alcalin ou un métal alcalino-terreux, un groupe ammonium substitué ou non substitué ou des groupes basiques organiques.

2. Mélanges selon la revendication 1, **caractérisés en ce qu'**il s'agit pour les groupes organiques basiques sous la définition de X et de Y d'aminoacides organiques basiques, comme l'arginine, l'ornithrine, la lysine, et les alcanolamines.

3. Mélanges selon les revendications 1 et/ou 2, **caractérisés en ce qu'**ils contiennent les monoesters de formule 1 et les diesters de formule II en quantités de 30 à 60, en particulier de 40 à 50 % en poids.

4. Mélanges selon une ou plusieurs des revendications 1 à 3, **caractérisés en ce qu'**ils contiennent les triesters de formule III en quantités comprises entre 0,5 et 5, de préférence entre 1 et 2 % en poids.

5. Formulations cosmétiques et/ou pharmaceutiques, **caractérisées en ce qu'**elles contiennent des mélanges selon une ou plusieurs des revendications 1 à 5 en quantités de 0,1 à 5, de préférence de 0,3 à 3 % en poids, par rapport au poids de la formulation.

6. Utilisation des mélanges selon une ou plusieurs des revendications 1 à 5 comme agent émulsionnant de produits cosmétiques et pharmaceutiques.

7. Utilisation des mélanges selon une ou plusieurs des revendications 1 à 5 en même temps que des agents co-émulsionnants.

8. Utilisation des mélanges selon une ou plusieurs des revendications 1 à 5 comme agent émulsionnant pour des émulsions d'huile-dans-l'eau.

9. Utilisation des mélanges selon une ou plusieurs des revendications 1 à 5 comme agent émulsionnant dans des produits de soins capillaires.
